# EUROPÄISCHE PATENTSCHRIFT

(11) **EP 3 633 354 B1**
(45) Veröffentlichungstag und Bekanntmachung des Hinweises auf die Patenterteilung: **15.03.2023**
(21) Anmeldenummer: 19212233.1
(22) Anmeldetag: 04.05.2016
(51) Int. Cl.: G01N 21/898, G01N 21/94, D06F 34/18, D06F 95/00, D06F 103/02

(54) **VERFAHREN ZUR PRÜFUNG GEWASCHENER ODER GEREINIGTER WÄSCHESTÜCKE**
METHOD FOR TESTING WASHED OR CLEANED LAUNDRY
PROCÉDÉ DE VÉRIFICATION DE LINGE LAVÉ OU NETTOYÉ

(30) Priorität: 01.06.2015 DE 102015006765
(43) Veröffentlichungstag der Anmeldung: 08.04.2020
(62) Teilanmeldung aus: 16001008.8
(73) Patentinhaber: Herbert Kannegiesser GmbH, 32602 Vlotho (DE)
(72) Erfinder: Bringewatt, Wilhelm, 32457 Porta Westfalica (DE); Heinz, Engelbert, 32602 Vlotho (DE)
(74) Vertreter: Möller, Friedrich

(56) Entgegenhaltungen:
- EP-A1- 2 573 550
- WO-A1-96/09533
- DE-A1- 3 242 447
- DE-U1- 20 105 840
- JP-A- H07 174 714
- US-A- 4 952 062
- US-A- 5 130 559

## Beschreibung

Die Erfindung betrifft ein Verfahren zur Prüfung gewaschener oder gereinigter Wäschestücke gemäß dem Oberbegriff des Anspruchs 1.

Bei der Reinigung bzw. beim Waschen von Wäschestücken aller Art, vor allem Flachwäsche und Formteile (z. B. Bekleidungsstücke), kann es vorkommen, dass nicht alle Flecken aus den verschmutzten Wäschestücken entfernt werden konnten, Löcher vorhanden sind und/oder die Wäschestücke noch andere Verunreinigungen oder Anhaftungen, wie vor allem Haare oder Fasern, aufweisen. Vor allem an den gewaschenen bzw. gereinigten Wäschestücken anhaftende Haare sind unakzeptabel. Solche Wäschestücke können nicht an Kunden zurückgeliefert werden.

Entscheidend ist auch insbesondere bei sogenannter Weißwäsche, vor allem wenn es sich dabei um Tischwäsche handelt, der Weißegrad.

Es ist aus der EP 2 573 550 A1 bekannt, Verunreinigungen, wie insbesondere Flecken und auch Risse, durch eine Kamera auf einer Seite oder auch mehrere Kameras auf gegenüberliegenden oder der gleichen Seite der Wäschestücke zu ermitteln. Das geschieht mit getrennten Prüfungen der Wäschestücke, um auf beiden Seiten Verunreinigungen einerseits und Risse andererseits feststellen zu können.

Aus der JP H 07174714 A ist es bekannt, Wäschestücke zwischen einem Trockner und einer nachfolgenden Faltmaschine auf Gewebefehler zu prüfen. Dazu wird das Wäschestück beleuchtet und von einem Linienbildsensor ein Bild über die gesamte Breite des Wäschestücks aufgenommen. Aus der Aufnahme werden Rückschlüsse auf Defekte des Wäschestücks wie Flecken oder Risse gezogen. Anhand des Helligkeitsgrads oder der Dunkelheit kann die Art des Fehlers des geprüften Wäschestücks festgestellt werden.

Aus der DE 201 05 840 U1 ist es bekannt, Stoffbahnen auf Fehler zu kontrollieren. Die jeweilige Stoffbahn wird von Transportbändern an einer Lichtquelle vorbeitransportiert. Das vom Wäschestück reflektierte Licht der Lichtquelle wird von einem Detektor ausgewertet, und zwar so, dass ein Rückschluss auf das reflektierende Material erfolgen kann.

Der Erfindung liegt die Aufgabe zugrunde, Verfahren zur Prüfung gewaschener oder gereinigter Wäschestücke zu schaffen, die auf einfache Weise eine umfassende Prüfung zulassen.

Ein Verfahren zur Lösung der eingangs genannten Aufgabe weist die Merkmale des Anspruchs 1 auf. Bei diesem Verfahren ist es vorgesehen, dass die Gestalt und/oder Umrisse von Verunreinigungen, wie Flecken, Anhaftungen, Muster, wie zum Beispiel Streifen, Ornamente, Embleme, Markierungen oder dergleichen, ermittelt und diese hinsichtlich ihrer Geometrie ausgewertet werden. Dadurch sind Rückschlüsse auf die Art der Verunreinigung, Anhaftungen oder des Musters möglich. Dazu ist es vorgesehen, ein Längen- und Breitenverhältnis der ermittelten Gestalt oder des Umrisses der jeweiligen Verunreinigung zu ermitteln. Anhand des Überschreitens eines bestimmten, vorgegebenen Längen- und Breitenverhältnisses kann die Gestalt der ermittelten Verunreinigung, beispielsweise ein Haar oder eine Faser, identifiziert werden. Vor allem Haare werden auf gewaschenen und gereinigten Wäschestücken als sehr unangenehm empfunden. Deswegen ist es wichtig, solche Haare zuverlässig identifizieren zu können und im Falle von im oder am Wäschestück vorhandenen Haaren dieses nicht an Kunden auszuliefern, sondern es nachzubehandeln oder erneut zu waschen oder zu reinigen.

Es kann vorgesehen sein, dass eine der mindestens einen bildgebenden Einrichtung, beispielsweise einer Kamera oder Sensoren, gegenüberliegende Seite des jeweiligen Wäschestücks beleuchtet, angestrahlt und/oder durchleuchtet wird. Dadurch erfolgt ein Hinterleuchten des jeweiligen Wäschestücks. Durch das Hinterleuchten und/oder Beleuchten der betreffenden Seite scheinen Verunreinigungen und/oder Anhaftungen dieser Seite des jeweiligen Wäschestücks auf die gegenüberliegende Seite, der die mindestens eine Kamera oder der mindestens eine Sensor zugeordnet sind, durch. Auf der der beleuchteten Seite des Wäschestücks gegenüberliegenden Seite werden dadurch Verunreinigungen und Anhaftungen der anderen, beleuchteten Seite des Wäschestücks für die mindestens eine Kamera oder den mindestens einen Sensor sichtbar.

Durch das Hinterleuchten des jeweiligen Wäschestücks scheinen Flecken oder sonstige Verunreinigungen und auch Anhaftungen, wie zum Beispiel Haare, von der angestrahlten Seite, vorzugsweise der Unter- bzw. Rückseite, deutlich durch das jeweilige Wäschestück hindurch, und zwar auch durch ein mehrlagiges Wäschestück. Dadurch können auch Verunreinigungen und/oder Anhaftungen der von der Kamera oder Sensoren nicht direkt zugänglichen Seite des jeweiligen Wäschestücks von der mindestens einen Kamera oder dergleichen auf der von diesem zugänglichen gegenüberliegenden Seite, insbesondere Vorderseite, des Wäschestücks zuverlässig erfasst werden.

Das erfindungsgemäße Verfahren ermöglicht es, Verunreinigungen und Anhaftungen auf beiden Seiten des Wäschestücks von mindestens einer Kamera oder einem Sensor oder einer sonstigen bildgebenden Einrichtung an nur einer Seite, insbesondere der Vorderseite, des Wäschestücks zu detektieren und auszuwerten. Es werden Verunreinigungen und/oder Anhaftungen auf der einen Seite, vorzugsweise der Unter- bzw. Rückseite, an der gegenüberliegenden Seite, insbesondere der Ober- bzw. Vorderseite, sichtbar gemacht, so dass diese auf beiden Seiten des Wäschestücks von einer Kamera oder sonstigen bildgebenden Einrichtungen auf nur einer Seite des jeweiligen Wäschestücks festgestellt werden können.

Bevorzugt wird eine solche Seite des Wäschestücks beleuchtet, angeleuchtet und/oder hinterleuchtet, bei der es sich um die Nutzseite, die in der Regel von der Finishseite des Wäschestücks gebildet wird, handelt. Dies ist die von den Benutzern später sichtbare Seite. Deswegen ist es insbesondere wichtig, dass diese Seite, die Finishseite, fleckenfrei ist. Die Nutz- bzw. Finishseite befindet sich nach dem Mangeln an der Rückseite des jeweiligen Wäschestücks. Das Beleuchten bzw. Durchleuchten des Wäschestücks an der dem mindestens einen Sensor, Scanner oder der mindestens einen Kamera gegenüberliegenden Seite lässt Verunreinigungen und/oder Anhaftungen an der Rückseite durch das Wäschestück hindurchscheinen, wodurch Verunreinigungen der Rückseite des Wäschestücks von der mindestens einen bildgebenden Einrichtung aufgenommen werden können, vorzugsweise zusammen mit Verunreinigungen und Anhaftungen auf der Vorderseite.

Gemäß einer vorteilhaften Ausgestaltungsmöglichkeit des Verfahrens findet die Prüfung bei mit der Nutzseite und/oder Finishseite ausgebreitet auf mindestens einem Förderer liegenden Wäschestück statt, wobei die auf dem Förderer aufliegende Nutz- und/oder Finishseite vorzugsweise von unten angeleuchtet, hinterleuchtet bzw. beleuchtet wird. Dadurch kommt es zu einem Durchleuchten des Wäschestücks, wodurch Flecken oder sonstige Verunreinigungen einschließlich Anhaftungen auf der Seite, die von der Kamera oder dergleichen weggerichtet ist, vorzugsweise der Rückseite, auch detektiert werden können. Es sind so Flecken auf beiden Seiten des Wäschestücks ermittelbar, obwohl die Flecken auf der stets unten auf dem Förderer liegenden Nutz- bzw. Finishseite von der Kamera, dem Scanner bzw. dem mindestens einen Sensor weggerichtet ist, weil sie auf der Seite des Förderers liegen.

Eine weitere vorteilhafte Ausgestaltungsmöglichkeit des Verfahrens sieht es vor, dass die der mindestens einen Kamera bzw. dem mindestens einen Scanner gegenüberliegende Seite, vorzugsweise die Nutz- und/oder Finishseite des Wäschestücks, gleichmäßig und/oder durchgehend über die ganze Breite beleuchtet, vorzugsweise ausgeleuchtet und/oder durchleuchtet, wird. Dadurch werden alle Flecken, Fremdkörper und/oder Beschädigungen auch auf der dem Scanner oder der Kamera gegenüberliegenden Seite sichtbar gemacht, so dass auch diese von der mindestens einen Kamera oder dem mindestens einen Scanner sichtbar und so zuverlässig erfassbar sind.

Insbesondere ist es vorgesehen, dass das jeweilige Wäschestück von der der mindestens einen Kamera oder dem mindestens einen Scanner gegenüberliegenden Seite, vorzugsweise der Nutz- oder Finishseite, derart beleuchtet bzw. hinterleuchtet wird, dass das Wäschestück durchleuchtet wird. Das Durchleuchten des Wäschestücks lässt alle Flecken oder sonstige Verunreinigungen, Beschädigungen (Löcher) und auch Anhaftungen dieser Seite auf der gegenüberliegenden Seite, auf der sich die mindestens eine Kamera oder dergleichen befinden, sichtbar werden. Auch Flecken oder sonstige Verunreinigungen bzw. Anhaftungen auf der verdeckten Rück- oder Unterseite des Wäschestücks, der keine Kamera oder dergleichen zugeordnet sind oder zugeordnet werden können, weil diese Seite beispielsweise auf einem Förderer liegt, können so ohne ein Umdrehen des Wäschestücks ermittelt werden.

Eine andere Ausgestaltungsmöglichkeit des Verfahrens sieht es vor, das Wäschestück von einer der Kamera oder Ähnlichem weggerichteten Seite derart anzuleuchten und/oder zu hinterleuchten, wodurch es zu einem Durchleuchten des jeweiligen Wäschestücks kommt. Dadurch werden mindestens Verunreinigungen und Anhaftungen auf der hinterleuchteten bzw. angestrahlten Seite auf der zur mindestens einen Kamera weisenden gegenüberliegenden Seite des Wäschestücks sichtbar. Es erscheinen Flecken, Anhaftungen, Beschädigungen und sonstige Beeinträchtigungen beider Seiten des Wäschestücks auf derjenigen Seite, worauf die mindestens eine Kamera oder Ähnliches gerichtet ist.

Eine Weiterbildungsmöglichkeit des Verfahrens sieht es vor, dass beide Seiten des jeweiligen Wäschestücks beleuchtet werden. Es wird also nicht nur die von der Kamera oder dem Sensor weggerichteten Seite des jeweiligen Wäschestücks beleuchtet, sondern auch von der gegenüberliegenden Seite, auf die die Kamera bzw. der Sensor gerichtet sind. Die beidseitige Beleuchtung und/oder Durchleuchtung des Wäschestücks ist besonders vorteilhaft bei einem mehrlagigen Wäschestück. Verunreinigungen, Anhaftungen oder auch Muster des Wäschestücks erscheinen so für die Kamera oder Sensoren kontrastreicher, was die Erkennung von Flecken, Haaren, Mustern oder dergleichen auf beiden Seiten des jeweiligen Wäschestücks vereinfacht und zuverlässiger gestaltet und auch Löcher besser erkennbar macht.

Gemäß einer bevorzugten Weiterbildung des Verfahrens wird mindestens eine Seite des jeweiligen Wäschestücks einer Strahlung ausgesetzt. Anhand der reflektierten Strahlung werden Rückschlüsse auf den Helligkeits- bzw. Weißegrad des jeweiligen Wäschestücks gezogen. Hellere, insbesondere weißere, Wäsche reflektiert die Strahlung anders. Insbesondere reflektieren weiße Wäsche oder helle Wäsche mehr Strahlung, so dass die Intensität der reflektierten Strahlung Rückschlüsse auf den Helligkeits- bzw. Weißegrad der Wäschestücke zulässt. Dieses Verfahren eignet sich besonders für weiße Wäsche, aber auch für helle Buntwäsche.

Bevorzugt wird der Weißegrad der Wäschestücke durch Vergleich der reflektierten Strahlung, vorzugsweise der Intensität der reflektierten Strahlung, mit Referenzwerten und/oder einem Referenzwertespektrum ermittelt. Die Referenzwerte oder das Referenzwertespektrum können in einer Steuerung oder einem Rechner hinterlegt sein. Die Werte sind gestaffelt nach größerem und kleinerem Weißegrad. Dementsprechend kann durch Vergleich des am jeweiligen Wäschestück ermittelten Werts der Intensität der reflektierten Strahlung mit den Werten aus dem Referenzwertespektrum festgestellt werden, in welchen Bereich des Referenzwertespektrums der aktuell am Wäschestück ermittelte Wert fällt. Ein höherer Wert, der einer größeren Reflexion der Strahlung entspricht, deutet auf einen größeren Weißegrad hin als ein geringerer Wert.

Bei der Strahlung kann es sich um verschiedenartige sicht- oder auch unsichtbare Strahlen handeln, vorzugsweise Lichtstrahlen, Ultraviolettstrahlen, Infrarotstrahlen oder dergleichen.

Bei einer bevorzugten Ausgestaltung des Verfahrens ist es vorgesehen, die Wäschestücke mit einer ultravioletten Strahlung (UV-Strahlung) zur Ermittlung des Weißegrads zu beaufschlagen. Der Umfang und die Intensität reflektierter UV-Strahlung ist ein zuverlässiger Indikator für den Weißegrad der Wäschestücke, so dass dieser besonders gut reproduzierbar ist und vor allem mit Referenzwerten gut vergleichbare Ergebnisse liefert.

Bevorzugte Ausführungsbeispiele der Erfindung werden nachfolgend anhand der Zeichnung näher erläutert. In dieser zeigen:
- Fig. 1: eine schematische Seitenansicht einer Vorrichtung zur Durchführung des erfindungsgemäßen Verfahrens mit einem Förderer zwischen einer Mangel und einer darauf folgenden Faltmaschine, und
- Fig. 2: eine schematische Seitenansicht einer alternativen Vorrichtung zur Durchführung des erfindungsgemäßen Verfahrens.

Bei den gezeigten Ausführungsbeispielen finden die erfindungsgemäßen Verfahren zwischen einer Mangel 10 und einer Faltmaschine 11 statt. Jedes in den Figuren von der Seite gezeigtes gemangelte Wäschestück 12 wird nach dem Verlassen eines Auslaufbereichs 13 der Mangel 10 von einem Förderer in Behandlungsrichtung 15 zur Faltmaschine 11 transportiert. In der Faltmaschine 11 wird das gemangelte Wäschestück 12 automatisch gefaltet.

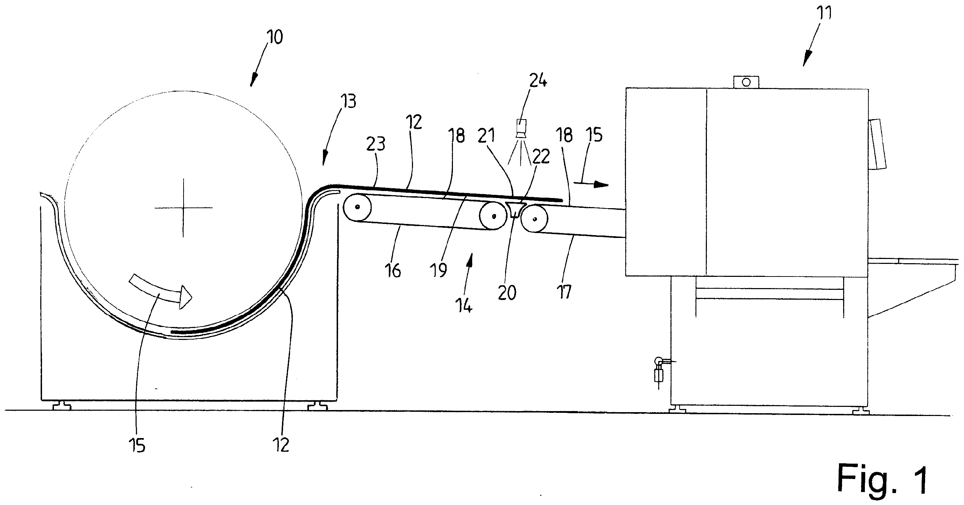

Im Ausführungsbeispiel der Fig. 1 ist der Förderer 14 von zwei aufeinanderfolgenden Förderern gebildet, und zwar einem Auslaufförderer 16 hinter dem Auslaufbereich 13 der Mangel 10 und einem sich daran anschließenden Einlaufförderer 17 vor der Faltmaschine 11. Der Auslaufförderer 16 und der Einlaufförderer 17 sind gleichermaßen als Gurtförderer mit einem umlaufenden Gurttrieb ausgebildet. Der Auslaufförderer 16 und der Einlaufförderer 17 können einen über die gesamte Arbeitsbreite durchgehenden Fördergurt oder auch mehrere nebeneinanderliegende schmale Fördergurte aufweisen. Über die Obertrume 18 des Auslaufförderers 16 und des Einlaufförderers 17 werden gemangelte und dadurch ausgebreitete, vorzugsweise flach ausgestreckte, Wäschestücke 12 ohne Überlappung, vorzugsweise mit Abstand zueinander, aufeinanderfolgend in Behandlungsrichtung 15 zur Faltmaschine 11 transportiert. Bei mehrbahniger Betriebsweise können auch die Wäschestücke 12 in mehreren Bahnen nebeneinanderliegend auf dem Förderer 16, 17 liegen. Gegebenenfalls können die Obertrume 18 perforiert sein, um mit Unterdruck die ausgebreiteten Wäschestücke 12 zumindest bereichsweise auf dem Auslaufförderer 16 bzw. dem Einlaufförderer 17 zu fixieren. Alternativ ist es auch denkbar, den Auslaufförderer 16 und den Einlaufförderer 17 mit netzartigen Gurten zu versehen. Die Wäschestücke 12 liegen dann auf dem netz- oder gitterartigen Obertrum 18 auf. Dadurch wird eine auf dem Förderer 14 aufliegende Unterseite jedes Wäschestücks 12 vom Förderergurt des Förderers 14, insbesondere des Auslaufförderers 16 und des Einlaufförderers 17, nahezu vollständig freigelassen.

Die Wäschestücke 12 verlassen die Mangel 10 mit unten liegender Finishseite 19, weil sich üblicherweise die Finishseite 19 der Wäschestücke 12 an der unteren stillstehenden Mangelmulde bildet. Mangeln anderer Bauart, zum Beispiel Bändermangeln, können die Wäschestücke 12 auch mit obenliegender Finishseite 19 verlassen. Deshalb ist die Erfindung nicht nur auf Wäschestücken 12 mit untenliegender Finishseite 19 beschränkt.

Die Finishseite 19 ist die spätere Nutzseite der Wäschestücke 12, und zwar bei Tischwäsche die Oberseite. Weil die die spätere Oberseite der Wäschestücke 12 bildende Finishseite 19 beim Verlassen der Mangel 10 unten liegt und damit auf dem Obertrum 18 des auf die Mangel 10 folgenden Förderers 14 bzw. Auslaufförderer 16 und Einlaufförderer 17, sind Muster, Löcher und Verunreinigungen, insbesondere Flecken, bei untenliegender Finishseite 19 des Wäschestücks 10 schwer ermittelbar. Bislang musste das jeweilige Wäschestück 12 dazu gewendet werden.

Erfindungsgemäß ist zwischen der Mangel 10 und der Faltmaschine 11 mindestens eine Leuchteinrichtung 20 vorgesehen. Die Leuchteinrichtung 20 ist derart zwischen der Mangel 10 und der Faltmaschine 11 angeordnet, dass sie die Wäschestücke 12 von unten, vorzugsweise ihren untenliegenden Finishseiten 19, her beleuchtet oder anleuchtet, wodurch es zu einem Hinterleuchten und/oder Durchleuchten der Wäschestücke 12 kommt. Dementsprechend ist die Leuchtstärke der Leuchteinrichtung 20 so bemessen, dass Löcher, Muster, Anhaftungen und Verunreinigungen, vor allem Flecken, an der Unterseite des jeweiligen Wäschestücks 12 zur oberen, freiliegenden Seite durchscheinen und dort auch sichtbar sind.

Die Leuchteinrichtung 20 beleuchtet einen quer zur Behandlungsrichtung 15 verlaufenden Querstreifen 21 jedes Wäschestücks 12. Zu diesem Zweck entspricht die Breite der Leuchteinrichtung 20 der Breite des Förderers 14, insbesondere des Auslaufbereichs 16 und des gleich breiten Einlaufbereichs 17.

Im Ausführungsbeispiel der Fig. 1 ist die Leuchteinrichtung 20 derart dem Förderer 14 zugeordnet, dass sie sich zwischen dem Auslaufförderer 16 und dem Einlaufförderer 17 befindet. Dabei überbrückt die Leuchteinrichtung 20 einen Spalt zwischen dem Auslaufförderer 16 und dem Einlaufförderer 17, befindet sich nämlich in einem von den angrenzenden Umlenk- bzw. Antriebstrommeln des Auslaufförderers 16 und des Einlaufförderers 17 liegenden oberen Keilbereich des Förderers 14. Dazu ist an diesen oberen Keilbereich des Förderers 14 der Querschnitt der Leuchteinrichtung 20 angepasst, so dass eine im gezeigten Ausführungsbeispiel ebene Oberseite 22 eines Gehäuses der Leuchteinrichtung 20 sich etwa in der Ebene des Obertrums 18 des Förderers 14, insbesondere des Auslaufförderers 16 und des Einlaufförderers 17, befindet. Im Gehäuse der Leuchteinrichtung 20 können die Leuchtmittel, vorzugsweise LED-Leuchtmittel, untergebracht sein, und zwar so, dass von den Leuchtmitteln ausgehende Strahlen nach oben durch die Oberseite 22 des Gehäuses hindurch gegen die Unterseite des Wäschestücks 12 gerichtet sind. Es ist auch denkbar, Leuchtstreifen mit darauf angeordneten kleinen LED-Leuchtmitteln auf die Oberseite 22 des Gehäuses aufzukleben. Dabei sind die LED-Leuchtmittel wiederum so orientiert, dass die von diesen abgesendeten Lichtstrahlen gegen die Unterseite des Wäschestücks 12 gerichtet und auf die Unterseite, vorzugsweise die Finishseite 19, des Wäschestücks 12 auftreffen.

Der dem Förderer 14 gegenüberliegenden Seite, insbesondere einer Oberseite 23 des Wäschestücks 12, ist eine bildgebende Einrichtung zugeordnet, die im gezeigten Ausführungsbeispiel als mindestens eine Kamera 24 ausgebildet ist. Die Kamera 24 ist mit Abstand über dem Wäschestück 12 ortsfest positioniert. Diese Positionierung der Kamera 24 erfolgt derart, dass die Kamera 24 die Oberseite 23 des Wäschestücks 12 vorzugsweise vollflächig abbildet. Dabei ist die Kamera 24 so ausgebildet, dass sie zumindest den von der Leuchteinrichtung 20 gleichmäßig und vollständig, vor allem lückenlos, hinterleuchteten, angeleuchteten und/oder durchleuchteten Querstreifen 21 des Wäschestücks 12 über die gesamte Breite desselben erfasst und hiervon ein Bild mit den Verunreinigungen, Löchern, Mustern oder dergleichen auf der unteren, durchleuchteten Finishseite 19 und vorzugsweise auch zugleich der Oberseite 23 erzeugt.

Erforderlichenfalls kann auf der Seite der Kamera 24 auch noch mindestens eine Leuchtquelle vorgesehen sein. Diese Leuchtquelle kann gegebenenfalls in die Kamera 24 integriert sein. Die mindestens eine Leuchtquelle auf der Seite der Kamera 24, also der freiliegenden Oberseite des Wäschestücks 12, leuchtet vorzugsweise auch einen Querstreifen 21 des Wäschestücks 12 über die gesamte Breite aus. Auch bei dieser mindestens einen Lichtquelle kann es sich um eine Reihe mehrerer LED-Leuchtmittel handeln. Diese Leuchtmittel erzeugen ein auf die freiliegende Oberseite des Wäschestücks 12 gerichtetes Auflicht. In diesem Fall wird das Wäschestück im Bereich des Querstreifens 21 beidseitig beleuchtet, und zwar von der freiliegenden Oberseite mit einem Auflicht und auf der gegenüberliegenden, dem Förderer 14 zugewandten Unterseite mit Licht hinter- und durchleuchtet. Die doppelseitige Beleuchtung, insbesondere das An- und Durchleuchten des jeweiligen Wäschestücks 12, erleichtert und verbessert die Erkennung von Beeinträchtigungen des jeweiligen Wäschestücks 12, insbesondere Flecken, Löcher oder dergleichen. Die beidseitige Be- und Durchleuchtung des jeweiligen Wäschestücks 12 lässt auch eine bessere und zuverlässige Erkennung von Mustern des Wäschestücks 12 oder Fremdkörpern auf demselben, beispielsweise Haare, zu.

Es ist denkbar, mehrere Kameras 24 oder sonstige bildgebende Einrichtungen vorzusehen, insbesondere in einer quer zur Behandlungsrichtung 15 verlaufenden Reihe. Alternativ könnten an der Stelle der Kamera 24 auch lichtsensitive Sensoren vorgesehen sein, vorzugsweise mehrere Sensoren nach Art eines Scanners in einer quer zur Behandlungsrichtung 15 verlaufenden Reihe mit gleichmäßigem Abstand nebeneinander angeordnet sein, so dass sie die gesamte Breite des Wäschestücks 12 bzw. die Breite mehrerer nebeneinanderliegender Wäschestücke 12, lückenlos abscannen.

Die mindestens eine Kamera 24 ist bevorzugt mit einer selbst-fokussierenden Optik versehen. Diese ermöglicht eine automatische Scharfeinstellung. Dadurch können zum Beispiel auch Beeinträchtigungen oder Fremdkörper an einem Wäschestück 12 erkannt werden, das im Bereich der mindestens einen Kamera 24 nicht flach auf dem Förderer 14 aufliegt, sondern etwas aufgewölbt ist. Außerdem ist die automatische Scharfeinstellung der mindestens einen Kamera 14 in der Lage, erforderlichenfalls eine Verstellung derart vorzunehmen, dass zum Beispiel Flecken auf der zur Kamera 24 weisenden Oberseite des Wäschestücks 12 und/oder von der von unten angeleuchteten Unterseite des Wäschestücks 12 durchscheinende Flecken oder dergleichen scharf erscheinen. Die automatische Einstellung der Optik der mindestens einen Kamera 24 kann auch in Abhängigkeit davon erfolgen, was sie momentan aufnimmt, beispielsweise einen Fleck, ein Loch, ein Muster oder dergleichen.

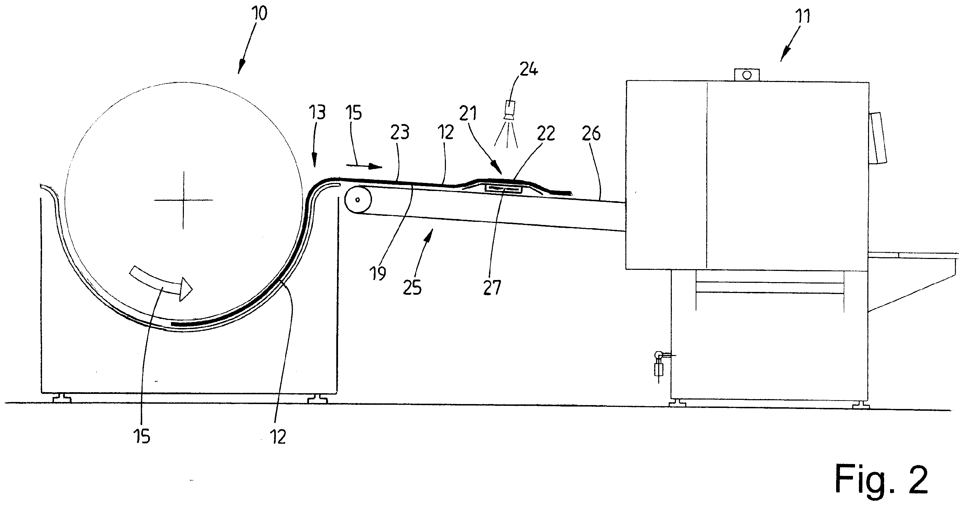

Die Fig. 2 zeigt ein alternatives Ausführungsbeispiel mit einem einzigen durchgehenden Förderer 25 zwischen der Mangel 10 und der Faltmaschine 11. Dicht über einem Obertrum 26 dieses Förderers 25 ist eine über die gesamte Breite des Förderers 25 quer zur Behandlungsrichtung 15 durchgehende Leuchteinrichtung 27 angeordnet. Das Obertrum 26 ist unter der Leuchteinrichtung 27 hinweggeführt, wobei entsprechende Leitbleche im Bereich der Leuchteinrichtung 27 das Wäschestück 12 vom Obertrum 26 ablenken, insbesondere abheben, so dass das Wäschestück 12 im Bereich der Leuchteinrichtung 27 über denselben hinweg transportiert wird. Dadurch wird die Leuchteinrichtung 27 nicht vom Obertrum 26 überdeckt, so dass die Leuchteinrichtung 27 direkt mit der Unterseite, insbesondere Finishseite 19, des Wäschestücks 12 in Kontakt kommt und dieses von unten anleuchten und/oder durchleuchten kann, so dass Flecken, Löcher oder dergleichen an bzw. in der Unterseite des Wäschestücks 12 zur Oberseite desselben durchscheinen und so auch von der mindestens einen der Oberseite des Wäschestücks 12 zugeordneten Kamera 24 erfassbar sind. Falls das Obertrum 27 über die Leuchteinrichtung 27 hinweggeführt wird, ist das Obertrum 26 des Förderers 14 entweder lichtdurchlässig ausgebildet oder es ist netz- oder gitterartig ausgebildet und dadurch nahezu vollständig lichtdurchlässig.

Abweichend vom Ausführungsbeispiel der Fig. 2 kann eine flach ausgebildete Leuchteinrichtung 27 auch zwischen den Trumen des Förderers 25 angeordnet sein, so dass die mit der Unterseite, insbesondere Finishseite 19, des jeweiligen Wäschestücks 12 in Kontakt kommende Oberseite 22 der Leuchteinrichtung 27 in der Hauptebene des Obertrums 26 (vor und hinter der Leuchteinrichtung 27) liegt und dadurch die Leuchteinrichtung 27 im Gegensatz zum Ausführungsbeispiel der Fig. 2 keine Erhöhung über dem Obertrum 26 des Förderers 25 bildet.

Die Leuchteinrichtung 27 kann wie die im vorangehenden Ausführungsbeispiel näher beschriebene Leuchteinrichtung 20 ausgebildet sein. Zur Vermeidung von Wiederholungen wird auf die Beschreibung der Leuchteinrichtung 20 Bezug genommen.

Nachfolgend wird das erfindungsgemäße Verfahren anhand der Vorrichtung der Fig. 1 näher erläutert:
Mit dem erfindungsgemäßen Verfahren ist es möglich, Verunreinigungen, insbesondere Flecken, aber auch Löcher, Anhaftungen, zum Beispiel Haare, und dergleichen, auf der Oberseite 23 und der Unterseite, insbesondere der Finishseite 19, von Wäschestücken 12 gleichzeitig zu ermitteln, ohne die Wäschestücke 12 wenden zu müssen. Die Ermittlung von Verunreinigungen, vor allem Flecken und Anhaftungen, und auch Löcher erfolgt kontinuierlich auf dem Weg des Wäschestücks 12 von der Mangel 10 zur Faltmaschine 11. Dadurch können die Verunreinigungen, Löcher und Anhaftungen am vorzugsweise flach und ausgestreift auf dem Obertrum 18 des Förderers 14 zwischen der Mangel 10 und der Faltmaschine 11 liegenden gemangelten Wäschestücks 12 detektiert werden.

Von der mindestens einen Kamera 24 oder einer anderen bildgebenden Einrichtung wird beim Vorbeilaufen kontinuierlich nach und nach jeweils ein Querstreifen 21 des Wäschestücks 12 mit durchscheinenden unteren Anhaftungen und Verunreinigungen und auch oberen Verunreinigungen und Anhaftungen sowie Löchern von der mindestens einen Kamera 24 aufgenommen. Dabei scannt die Kamera 24 nach und nach die gesamte Oberseite 23 des Wäschestücks vollflächig und erzeugt hiervon mindestens ein Bild.

Verunreinigungen, insbesondere Flecken, auf der Oberseite 23 des jeweiligen Wäschestücks 12 werden von der Kamera 24 direkt erfasst. Verunreinigungen, insbesondere Flecken, auf zum Obertrum 18 des Förderers 14 weisenden oder aufliegenden Unterseite bzw. Finishseite 19 des jeweiligen Wäschestücks 12 werden erfindungsgemäß auch von der Kamera 24 von der Oberseite 23 her ermittelt, indem von der mindestens einen Leuchteinrichtung 20 die Unterseite, insbesondere Finishseite 19, des jeweiligen Wäschestücks beleuchtet oder angestrahlt wird. Dies erfolgt derart, dass das Wäschestück 12 von der Finishseite 19 her durchleuchtet wird, wodurch Verunreinigungen, insbesondere Flecken, der Unterseite bzw. Finishseite 19 des jeweiligen Wäschestücks 12 durch das Wäschestück 12 hindurchscheinen und dadurch auch an der Oberseite 23 des Wäschestücks 12 von der Kamera 24 ermittelbar, vor allem abbildbar, sind. Auf diese Weise kann die mindestens eine nur einer Seite des Wäschestücks 12 zugeordnete Kamera 24 oder eine sonstige bildgebende Einrichtung nicht nur Löcher oder sonstige Beschädigungen, sondern auch Verunreinigungen sowohl auf der Oberseite 23 als auch auf der untenliegenden Seite, zum Beispiel der Finishseite 19, gleichzeitig ermitteln. Zu diesem Zweck sind die Leuchtmittel oder Leuchtquellen der Leuchteinrichtung 20 ausgebildet, um genügend Licht zu erzeugen, dass Flecken oder sonstige Verunreinigungen an der Unterseite bzw. Finishseite 19 des jeweiligen Wäschestücks 12 durch dasselbe hindurchscheinen und auf der Oberseite 23 für die mindestens eine Kamera 24 oder eine sonstige bildgebende Einrichtung sichtbar werden.

In der vorstehend beschriebenen Weise läuft prinzipiell genauso das Verfahren bei der Vorrichtung der Fig. 2 ab.

Es ist verfahrensgemäß weiterhin vorgesehen, dass von der mindestens einen Kamera 24 oder einer sonstigen bildgebenden Einrichtung bzw. Sensoren beim kontinuierlich hieran vorbeilaufenden Wäschestück 12 die Gestalt der Verunreinigung bzw. Anhaftung ermittelt und hinsichtlich ihrer Geometrie ausgewertet wird.

Es werden bevorzugt Längen- und Breitenabmessungen der Verunreinigungen ermittelt und hieraus ein Längen-Breitenverhältnis ermittelt. In einer elektronischen Auswerteinrichtung wird das ermittelte Längen-Breitenverhältnis verglichen mit einem vorgegebenen Längen-Breitenverhältnis. Wenn dieser Vergleich ergibt, dass die Verunreinigung das vorgegebene Längen- und Breitenverhältnis erreicht oder überschreitet, ist das ein Indiz für eine bestimmte Verunreinigung. Bevorzugt werden nach diesem Verfahren auf oder an dem jeweiligen Wäschestück sich befindende Haare oder Fasern mit einem relativ großen Längen-Breitenverhältnis ermittelt. Dieses großes Längen-Breitenverhältnis lässt zuverlässig Rückschlüsse auf Haare oder Fasern zu, wenn die Auswertung ergibt, dass dieses große Längen-Breitenverhältnis erreicht oder überschritten wird.

Die vorstehend geschilderte Bestimmung von Verunreinigungen mit zum Beispiel Haaren anhand der Ermittlung des Längen- und Breitenverhältnisses derselben kann auch auf beiden Seiten des Wäschestücks 12 erfolgen. Durch Anleuchten bzw. Hinterleuchten einer Unterseite des Wäschestücks 12 wird dieses durchleuchtet und dadurch quasi durchsichtig gemacht, so dass auch an der Unterseite des Wäschestücks 12 vorhandene Verunreinigungen und Anhaftungen, wie zum Beispiel Haare, durch das Wäschestück 12 durchscheinen und dadurch an der Oberseite desselben sichtbar werden. Es können somit Verunreinigungen und Anhaftungen auf beiden Seiten eines jeweiligen Wäschestücks 12 detektiert und bestimmt werden.

Verfahrensmäßig ist es weiterhin vorgesehen, mindestens eine Seite des Wäschestücks 12 beim Vorbeilaufen an der Kamera 24 einer Strahlung auszusetzen und die reflektierte Strahlung beispielsweise von der Kamera 24 oder auch einem anderen Sensor- oder Detektionsmittel, zu erfassen. Als Strahlung kommen insbesondere Lichtstrahlen, eine ultraviolette Strahlung und/oder eine Infrarotstrahlung in Betracht.

Die Intensität der ermittelten reflektierten Strahlung ist ein Indiz für den Weißegrad des Wäschestücks 12. Besonders hohe reflektierte Strahlung lässt auf ein sehr weißes oder helles Wäschestück 12 schließen. Durch Vergleich mit Referenzwerten oder einem Referenzwertspektrum kann so festgestellt werden, wie hell oder wie weiß das Wäschestück 12 ist. Falls dabei vorgegebene Richt- bzw. Grenzwerte überschritten werden, also das Wäschestück zu hell oder zu dunkel ist, erfolgt eine Einflussnahme auf den Waschprozess nachfolgender Wäschestücke 12, indem waschaktive Substanzen und/oder Bleichmittel hinsichtlich ihrer Dosierung entsprechend verändert werden.

Die Erfindung eignet sich auch für sogenannte Trockenwäsche, die nach dem Waschen ohne ein vorheriges Mangeln direkt auf den zur Faltmaschine 11 führenden Einlaufförderer 17 aufgelegt und von dort der Faltmaschine 11 zugeführt wird. Hier findet das zuvor beschriebene Verfahren dann im Bereich des Einlaufförderers 17 vor der Faltmaschine 11 statt.

Das erfindungsgemäße Verfahren kann auch an hängenden, insbesondere ausgestreckt hängenden, Wäschestücken 12 durchgeführt werden. Dann ist einer Seite des hängenden Wäschestücks 12 die mindestens eine Kamera 24 zugeordnet und der gegenüberliegenden Seite die Leuchteinrichtung 20. Verunreinigungen und/oder Anhaftungen, wie zum Beispiel Haare auf der Seite der Leuchteinrichtung 20, scheinen durch das Beleuchten von der Leuchteinrichtung 20 dann auf die gegenüberliegende, der Kamera 24 zugeordnete Seite des Wäschestücks 12 durch und werden dann auch auf der Seite der bildgebenden Einrichtung, wie zum Beispiel der Kamera 24, sichtbar. Dadurch werden auch bei hängenden Wäschestücken 12 von der Kamera 24 Verunreinigungen bzw. Anhaftungen auf beiden Seiten der Wäschestücke 12 aufgrund des Durchleuchtens derselben und das dadurch hervorgerufene Durchscheinen von Verunreinigungen und Anhaftungen auf der der Leuchteinrichtung 20 zugerichteten Seite der Wäschestücke 12 auf der gegenüberliegenden, zur Kamera 24 weisenden Seite der Wäschestücke 12. So werden auch Verunreinigungen beider Seiten der Wäschestücke 12 auf der der mindestens einen Kamera 24 zugerichteten Seite der Wäschestücke 12 für die mindestens eine Kamera 24 sichtbar und erfassbar.

Es ist eine elektronisch, automatische Auswertung, beispielsweise eine rechnergestützte Bildauswertung der von der mindestens einen Kamera 24, dem Sensor oder einer sonstigen bildgebenden Einrichtung erfassten Bilder vorgesehen. Gegebenenfalls kann die Bildauswertung die Struktur des Gewebes der Wäschestücke 12 ausfiltert. Dadurch ist sichergestellt, dass die Gewebestruktur der Wäschestücke 12 die Erkennung von Verunreinigungen (Flecken) und/oder Anhaftungen (Haare) nicht beeinträchtigt.

Weiterhin kann es vorgesehen sein, von der mindestens einen Kamera, Sensoren oder dergleichen das Wäschestück gleichzeitig zu identifizieren, so dass ermittelbar ist, um welches Wäschestück es sich handelt. Das ermöglicht eine Feststellung, ob am gleichen Wäschestück mehrfach die gleiche Beeinträchtigung, beispielsweise der gleiche Fleck oder das gleiche Loch, erkannt worden ist. Wird zum Beispiel ein gleicher Fleck mehrfach detektiert, deutet das darauf hin, dass dieser Fleck nicht entfernbar ist. Ein solches Wäschestück kann dann aussortiert werden und braucht nicht wiederholt gewaschen zu werden. Festgestellt werden kann auch, wie sich ein bestimmter Fleck beim wiederholten Waschen oder einer sonstigen Behandlung verändert. Wird keine Veränderung festgestellt, deutet das auf eine Beeinträchtigung, beispielsweise einen Fleck, hin, der nicht entfernbar ist. Wenn die Beeinträchtigung, beispielsweise der Fleck, aber bei wiederholtem Waschen abnimmt, kann durch ein mindestens einmal wiederholtes Waschen versucht werden, den Fleck weiter oder ganz zu entfernen.

### Bezugszeichenliste:

- 10: Mangel
- 11: Faltmaschine
- 12: Wäschestück
- 13: Auslaufbereich
- 14: Förderer
- 15: Behandlungsrichtung
- 16: Auslaufförderer
- 17: Einlaufförderer
- 18: Obertrum
- 19: Finishseite
- 20: Leuchteinrichtung
- 21: Querstreifen
- 22: Oberseite (von 20 und 27)
- 23: Oberseite
- 24: Kamera
- 25: Förderer
- 26: Obertrum
- 27: Leuchteinrichtung

## Patentansprüche

1. Verfahren zum Prüfen gewaschener oder gereinigter Wäschestücke (12), wobei Verunreinigungen, insbesondere Flecken, Haare oder Fasern und/oder Muster, durch mindestens eine auf wenigstens eine Seite des jeweiligen Wäschestücks (12) gerichtete Kamera (24) oder mindestens einen Sensor erfasst werden, **dadurch gekennzeichnet, dass** die Gestalt und/oder Umrisse der Verunreinigungen ermittelt und hinsichtlich ihrer Geometrie ausgewertet werden, indem ein Längen-Breitenverhältnis der ermittelten Gestalt der jeweiligen Verunreinigung ermittelt und mit einem bestimmten, vorgegebenen Längen-Breitenverhältnis verglichen wird, wobei ein Erreichen und Überschreiten des vorgegebenen Längen- und Breitenverhältnisses als eine bestimmte Verunreinigung, wie beispielsweise ein Haar oder eine Faser, gewertet wird.

2. Verfahren nach Anspruch 1, **dadurch gekennzeichnet, dass** eine einer solchen Seite, der die mindestens eine Kamera (24) oder der mindestens eine Sensor zugeordnet sind, gegenüberliegende Seite des Wäschestücks (12) angeleuchtet, beleuchtet und/oder durchleuchtet wird.

3. Verfahren nach Anspruch 1, **dadurch gekennzeichnet, dass** eine solche Seite des jeweiligen Wäschestücks (12) beleuchtet und/oder durchleuchtet wird, bei der es sich um die Finishseite (19) des Wäschestücks (12) handelt.

4. Verfahren nach einem der vorhergehenden Ansprüche, **dadurch gekennzeichnet, dass** Verunreinigungen, insbesondere Flecken, Haare oder dergleichen, an der beleuchteten bzw. angeleuchteten Seite des Wäschestücks (12) durch dasselbe hindurchscheinen und dadurch auf der gegenüberliegenden Seite des Wäschestücks (12), der die mindestens eine Kamera (24) oder der mindestens eine Sensor zugeordnet ist, sichtbar werden bzw. sichtbar gemacht werden.

5. Verfahren nach einem der vorhergehenden Ansprüche, **dadurch gekennzeichnet, dass** die Prüfung im mit der Nutzseite und/oder Finishseite (19) ausgebreitet auf einem Förderer (14, 25) liegenden Zustand des Wäschestücks (12) erfolgt, wobei die auf dem Förderer (14, 25) liegenden Nutz- und/oder Finishseite (19) vorzugsweise von unten beleuchtet, angeleuchtet und/oder durchleuchtet wird.

6. Verfahren nach Anspruch 5, **dadurch gekennzeichnet, dass** die der mindestens einen Kamera (24) bzw. dem mindestens einen Sensor gegenüberliegende Seite, vorzugsweise die Nutz- und/oder Finishseite (19), des Wäschestücks (12) gleichmäßig über die gesamte Breite des jeweiligen Wäschestücks (12) durchgehend, insbesondere im Bereich eines Querstreifens (21) des jeweiligen Wäschestücks (12), beleuchtet und/oder durchleuchtet wird, vorzugsweise kontinuierlich beim Weitertransport des jeweiligen Wäschestücks (12) vom Förderer (14, 25) in Behandlungsrichtung (15).

7. Verfahren nach einem der vorhergehenden Ansprüche, **dadurch gekennzeichnet, dass** die Wäschestücke (12) von der der mindestens einen Kamera (24) und/oder dem mindestens einen Sensor gegenüberliegenden Seite, vorzugsweise der Nutz- oder Finishseite (19), derart beleuchtet werden, dass sie durchleuchtet werden und/oder Verunreinigungen, insbesondere Flecken, auf der Nutz- oder Finishseite (19), durch die Wäschestücke (12) durchscheinen und so auch Verunreinigungen, Flecken bzw. Anhaftungen auf der Nutz- oder Finishseite (19) der Wäschestücke (12) auf der gegenüberliegenden Seite, der die mindestens eine Kamera (24) bzw. der mindestens eine Sensor zugeordnet ist, sichtbar sind.

8. Verfahren nach einem oder mehreren der Ansprüche 1 bis 7, **dadurch gekennzeichnet, dass** mindestens eine Seite des Wäschestücks (12) beim Vorbeilaufen an einer Kamera (24), einer sonstigen bildgebenden Einrichtung oder Sensoren einer Strahlung ausgesetzt wird und anhand der reflektierten Strahlung Rückschlüsse auf den Weißegrad des jeweiligen Wäschestücks (12) gezogen werden, indem die reflektierte Strahlung von der Kamera (24), einer sonstigen bildgebenden Einrichtung oder Sensoren erfasst wird.

9. Verfahren nach Anspruch 8, **dadurch gekennzeichnet, dass** durch Vergleich der reflektierten Strahlung, vorzugsweise Intensität der reflektierten Strahlung, mit Referenzwerten und/oder einem Referenzwertespektrum der Weißegrad des jeweiligen Wäschestücks (12) ermittelt wird.

10. Verfahren nach Anspruch 8 oder 9, **dadurch gekennzeichnet, dass** als Strahlung eine ultraviolette Strahlung verwendet wird.

## Claims

1. Method for examining washed or cleaned items of laundry (12), wherein contaminants, in particular stains, hair or fibres, and/or patterns are detected by at least one camera (24) directed onto one side of the respective item of laundry (12) or by at least one sensor, **characterized in that** the form and/or outlines of the contaminants is/are established and evaluated in respect of the geometry thereof by a length-to-width ratio of the established form of the respective contaminant being established and compared with a specific, predetermined length-to-width ratio, with reaching and exceeding the predetermined length and width ratio being evaluated as a specific contaminant such as for example a hair or a fibre.

2. Method according to Claim 1, **characterized in that** a side of the item of laundry (12) lying opposite such a side associated with the at least one camera (24) or the at least one sensor is shone upon, illuminated and/or transilluminated.

3. Method according to Claim 1, **characterized in that** such a side of the respective item of laundry (12) which is the finish side (19) of the item of laundry (12) is illuminated and/or transilluminated.

4. Method according to one of the preceding claims, **characterized in that** contaminants, in particular stains, air or the like, on the illuminated or shone-onto side of the item of laundry (12) can be seen through the same and thereby become visible or are made visible on the opposite side of the item of laundry (12) which is associated with the at least one camera (24) or the at least one sensor.

5. Method according to one of the preceding claims, **characterized in that** the examination is carried out in the state of the item of laundry (12) in which it is lying with the used side and/or finish side (19) spread out on a conveyor (14, 25), with the used and/or finish side (19) that is lying on the conveyor (14, 25) being illuminated, shone onto and/or transilluminated, preferably from below.

6. Method according to Claim 5, **characterized in that** the side of the item of laundry (12) that is lying opposite the at least one camera (24) or the at least one sensor, preferably the used and/or finish side (19), is illuminated and/or transilluminated in a uniform manner continuously over the entire width of the respective item of laundry (12), in particular in the region of a transverse strip (21) of the respective item of laundry (12), preferably continuously during the onward transport of the respective item of laundry (12) by the conveyor (14, 25) in the treatment direction (15).

7. Method according to one of the preceding claims, **characterized in that** the items of laundry (12) are illuminated from the side lying opposite the at least one camera (24) and/or the at least one sensor, preferably the used and/or finish side (19), in such a way that they are transilluminated and/or contaminants, in particular stains, on the used finish side (19) can be seen through the items of laundry (12) and hence contaminants, stains or adhesions on the used or finish side (19) of the items of laundry (12) are also visible on the opposite side which is associated with the at least one camera (24) or the at least one sensor.

8. Method according to one or more of Claims 1 to 7, **characterized in that** at least one side of the item of laundry (12) is exposed to radiation when it passes by a camera (24), any other imaging device or sensors and conclusions about the degree of whiteness of the respective item of laundry (12) are drawn on the basis of the reflected radiation by the reflected radiation being detected by the camera (24), other imaging device or sensors.

9. Method according to Claim 8, **characterized in that** the degree of whiteness of the respective item of laundry (12) is established by comparing the reflected radiation, preferably intensity of the reflected radiation, with reference values and/or a reference value spectrum.

10. Method according to Claim 8 or 9, **characterized in that** ultraviolet radiation is used as radiation.

## Revendications

1. Procédé permettant de contrôler des pièces de linge (12) lavées ou nettoyées, dans lequel des salissures, en particulier des taches, des cheveux ou des fibres et/ou des dessins, sont détectées par au moins une caméra (24) orientée sur au moins une face de la pièce de linge (12) respective ou par au moins un capteur,
**caractérisé en ce que** la forme et/ou les contours des salissures sont établis et analysés quant à leur géométrie **en ce qu'**un rapport longueur/largeur de la forme établie de la salissure respective est établi et comparé avec un rapport longueur/largeur prédéfini déterminé, dans lequel le fait d'atteindre ou de dépasser le rapport longueur/largeur prédéfini est considéré comme une salissure déterminée, comme par exemple un cheveu ou une fibre.

2. Procédé selon la revendication 1, **caractérisé en ce qu'**une face de la pièce de linge (12), opposée à la face à laquelle ladite au moins une caméra (24) ou ledit au moins un capteur est associé (e), est illuminée, éclairée et/ou mirée.

3. Procédé selon la revendication 1, **caractérisé en ce qu'**une face de la pièce de linge (12) respective qui est la face de finition (19) de la pièce de linge (12) est éclairée et/ou mirée.

4. Procédé selon l'une quelconque des revendications précédentes, **caractérisé en ce que** des salissures, en particulier des taches, des cheveux ou similaires, sur la face éclairée ou illuminée de la pièce de linge (12) apparaissent à travers celle-ci, et sont ainsi visibles ou rendues visibles sur la face opposée de la pièce de linge (12) à laquelle ladite au moins une caméra (24) ou ledit au moins un capteur est associé(e).

5. Procédé selon l'une quelconque des revendications précédentes, **caractérisé en ce que** le contrôle est effectué à l'état où la pièce de linge (12) est posée avec la face utile et/ou la face de finition (19) étalée sur un convoyeur (14, 25), dans lequel la face utile et/ou de finition (19) posée sur le convoyeur (14, 25) est de préférence éclairée, illuminée et/ou mirée par le bas.

6. Procédé selon la revendication 5, **caractérisé en ce que** la face opposée à ladite au moins une caméra (24) ou audit au moins un capteur, de préférence la face utile et/ou de finition (19), de la pièce de linge (12) est éclairée et/ou mirée sans interruption de façon homogène sur toute la largeur de la pièce de linge (12) respective, en particulier au niveau d'une bande transversale (21) de la pièce de linge (12) respective, de préférence en continu lors du transport ultérieur de la pièce de linge (12) respective du convoyeur (14, 25) dans la direction de traitement (15) .

7. Procédé selon l'une quelconque des revendications précédentes, **caractérisé en ce que** les pièces de linge (12) sont éclairées à partir de la face opposée à ladite au moins une caméra (24) et/ou audit au moins un capteur, de préférence la face utile ou de finition (19), de façon à être mirées et/ou que des salissures, en particulier des taches, sur la face utile ou de finition (19), apparaissent à travers les pièces de linge (12), et de ce fait, mêmes des salissures, des taches ou des adhérences sur la face utile ou de finition (19) des pièces de linge (12) sont visibles sur la face opposée à laquelle ladite au moins une caméra (24) ou ledit au moins un capteur est associé(e) .

8. Procédé selon une ou plusieurs des revendications 1 à 7, **caractérisé en ce qu'**au moins une face de la pièce de linge (12) est exposée à un rayonnement lorsqu'elle passe devant une caméra (24), un autre dispositif d'imagerie ou des capteurs, et des conclusions concernant la blancheur de la pièce de linge (12) respective sont tirées à l'aide du rayonnement réfléchi **en ce que** le rayonnement réfléchi est détecté par la caméra (24), un autre dispositif d'imagerie ou des capteurs.

9. Procédé selon la revendication 8, **caractérisé en ce que** la comparaison du rayonnement réfléchi, de préférence de l'intensité du rayonnement réfléchi, avec des valeurs de référence et/ou un spectre de valeurs de référence permet d'établir la blancheur de la pièce de linge (12) respective.

10. Procédé selon la revendication 8 ou 9, **caractérisé en ce qu'**un rayonnement ultraviolet est utilisé comme rayonnement.
